# EUROPEAN PATENT SPECIFICATION

(11) **EP 2 144 625 B1**
(45) Date of publication and mention of the grant of the patent: **09.02.2022**
(21) Application number: 07865205.4
(22) Date of filing: 05.12.2007
(51) Int. Cl.: A61K 38/43, A61K 47/32, A61P 17/00, A61K 9/06, A61K 38/48, A61K 47/10

(54) **A CONTROLLED RELEASE ENZYMATIC COMPOSITION AND METHODS OF USE**
ENZYMATISCHE ZUSAMMENSETZUNG MIT KONTROLLIERTER FREISETZUNG UND ANWENDUNGSVERFAHREN
COMPOSITION ENZYMATIQUE À LIBÉRATION CONTRÔLÉE ET PROCÉDÉS D'UTILISATION

(30) Priority: 05.12.2006 US 868656 P
(43) Date of publication of application: 20.01.2010
(60) Divisional application: 21217552.5
(73) Proprietor: Marizyme, Inc., Jupiter, FL 33458 (US)
(72) Inventor: DOLAK, Terence M., Andover, NJ 07821 (US); RUTMAN, Max, 7550154 Santiago (CL); VINCENT, Jan, 114 42 Stockholm (SE); HELLGREN, Kristian, 260 40 Viken (SE)
(74) Representative: Dr. Gassner & Partner mbB
(86) International application number: PCT/US2007/086455
(87) International publication number: WO 2008/070698

(56) References cited:
- WO-A2-01/82945
- WO-A2-03/000625
- JP-A- 01 283 213
- US-A- 5 356 800
- US-A- 5 830 449

## Description

### BACKGROUND OF THE INVENTION

### 1. Field of the Invention

The invention relates to a low water content, enzyme-containing gel compositions capable of controlling enzymatic release and enzymatic activity level. More specifically, the invention relates to a low water content, enzyme-containing gel composition including at least one proteolytic enzyme and the use of such compositions for wound treatment, skin treatment and various cleansing and anti-microbial applications.

### 2. Description of the Related Technology

Krill enzymes are known as a means for treating wounds, skin disorders and viral infections primarily because of their ability to rapidly degrade biological contaminants in alkaline, neutral or acid media. A method for effectively delivering the enzyme composition, controlling enzymatic release or increasing the enzymatic activity level, however, remains undisclosed.

U.S. Patent nos. 4,801,451 and 4,963,491 and International patent application publication no. WO 00/49991, each discloses a mixture of exopeptidases and endopeptidases isolated from a species of Antarctic krill, *Euphausia superba*, for use as a cleansing agent and as a means of debriding necrotic tissue. These patents, however, do not provide an effective means for controlling enzyme release, optimizing enzyme efficiency or maximizing enzymatic bioactivity.

U.S. Patent nos. 4,801,451 and 4,963,491; each disclose that a variety of additives and carriers may be incorporated in compositions containing krill enzymes. Suitable carriers may be sterile, aqueous and physiologically acceptable salt solutions, organic and inorganic gelforming materials, e.g. polymers, silicone oils and other substances, and mixtures thereof, providing the desired physical properties of the composition. Additives such as antimicrobials, perfumes, different anionic, cationic, zwitterionic and neutral surfactants (tensides and emulgators) are mentioned. These patents also indicate that such components are known for use in connection with other enzyme compositions for laundering or enzymatic debridement, see e.g. German patent application no. 2 130 833; British patent application nos. 1,280,497; 1,296,901 and 1,573,964; and U.S. Patent nos. 3,627,688; 3,855,142; 4,212,761; 4,243,546; 4,242,219; 4,287,082; 4,035,837 and 4,307,081.

Glycol and glycerol additives are mentioned in, for example, U.S. Patent nos. 3,627,688; 3,855,142 and 4,242,219. However, these patents do not appear to disclose that glycol or glycerol may be used to regulate enzyme activity, control enzyme release or improve enzyme bioavailability.

U.S. Patent nos. 6,030,612 and 6,524,814; and U.S. patent application publication nos. 2006/0134641 and 2005/0025722; each disclose compositions including multifunctional krill-derived enzymes and methods for using such enzymes in treating viral infections such as HIV and herpes, fungal infections and bacterial infections. These publications disclose formulation of krill enzymes in hydrogels and exemplify a number of hydrogel compositions. These publications also mention that for oral administration in capsule form, useful diluents are lactose and high molecular weight polyethylene glycols. Also mentioned are suppositories that are solid at room temperature and melt at body temperature, which may contain high molecular weight polyethylene glycols. These publications also mention that gels, analogous to the suppository formulation, can be used via vaginal, urethral and rectal administration.

Good wound healing is characterized by rapid and complete regeneration of the damaged tissue. Considerable effort has been expended in the study of wound dressings with the aim of finding which are most effective in promoting wound healing. The primary goal for management of necrotic wounds is to reduce the healing time. The first step toward this goal is the removal (debridement) of necrotic tissue. Debridement of necrotic tissue is important because necrotic tissue harbors bacteria and prevents healing. Selective debridement is preferable as it selectively removes necrotic tissue without removing healthy tissue.

Presently, selective debridement includes certain topical proteolytic enzyme preparations such as streptokinase-streptodornase, papain, collagenase, fibrinolysin-desoxyribonuclease, which dissolve or digest necrotic slough and eschar. In general, enzymatic debridement procedures dissolve only dead necrotic slough. Since the viable tissues are protected from enzymatic degradation by protease inhibitors; the living cells in the wound bed and around the ulcer area remain unaffected.

Recently there has been a trend to move from passive wound dressings to active wound dressings containing substances that have an effect both on cleansing and on the healing process. Advances in polymers produced the most significant changes in wound care dressings as factors such as water vapor and oxygen permeability, bacterial impermeability and selective absorption could be taken into account in new dressings, as well as being able to address specific requirements such as conformability, non-adherence and adhesiveness. This family of polymeric products includes foams, films, hydrogels and hydrocolloids. Wound dressings have also been combined with biodegradable carrier materials such as collagen, keratin, gelatin, carbohydrates or cellulose derivatives. Dressings have also been combined with numerous pharmacological and/or antibiotic compositions.

Proteolytic enzymes are currently used in ointments and sprays. They are typically active only for a limited period of time and consequently must be reapplied several times a day. For wound treatment, this would require frequent dressing changes, which is undesirable in view of the manpower required, patient discomfort and the frequent intrusions on the patient.

U.S. Patent no. 3,296,094 discloses aqueous solutions of proteolytic enzymes that retain their activity even after prolonged storage periods. These compositions are stabilized with a partially hydrolyzed and solubilized collagen and glycerol. The amount of glycerol required for stabilization in this composition is 35% to 60% by weight of the total composition.

U.S. Patent no. 5,830,449 discloses a composition for topical application containing at least one enzyme and at least one polyol, characterized in that the polyol is present in a quantity ranging from 30 to 99.99% by weight, relative to the total weight of the composition. Optionally, the composition comprises at least one structuring agent chosen from acrylic polymers, methacrylic polymers and oils. This composition is proposed for the purpose of preventing loss of activity of proteolytic enzymes when exposed to water over an extended period.

Krill-derived enzymes are unstable and susceptible to degradation in predominately water based media which results in decreased enzyme activity over time. Thus, there exists a need to reduce krill enzymatic decomposition in order to provide storage stable compositions containing krill enzymes, as well as to improve the effectiveness of krill enzyme-containing compositions.

### SUMMARY OF THE INVENTION

One embodiment of the present invention provides an enzyme containing gel composition and a polyol-containing diluent to provide controlled release of enzyme activity over time and/or extended duration of enzyme activity in various applications of the enzyme composition. The diluent component may optionally contain up to about 5% by weight of water.

Other embodiments of the invention provide methods of using enzyme containing gel compositions in wound care, skin care, treatment of diseases and viral infections, sterilization, and transplant assistance.

These and other objects, advantages and aspects of the invention will become apparent in light of the following detailed description of certain embodiments of the invention.

### BRIEF DESCRIPTION OF THE DRAWINGS

Figure 1 is a graph of enzyme activity versus time for gel solutions of krill enzymes.
Figures 2(a)-(c) are graphs of enzyme activity versus water content at Day 0 for gel compositions containing one of ethylene, propylene and butylene glycols.
Figures 3(a)-(c) are graphs of enzyme activity versus water content at Day 7 for enzymatic gel compositions containing one of ethylene, propylene and butylene glycols.
Figures 4(a)-(c) are graphs of enzyme activity versus water content at Day 14 for enzymatic gel compositions containing one of ethylene, propylene and butylene glycols.
Figures 5(a)-(c) are graphs of enzyme activity versus water content at Day 48 for enzymatic gel compositions containing one of ethylene, propylene and butylene glycols.
Figures 6(a)-(c) are graphs of enzyme activity versus water content at Day 56 for enzymatic gel compositions containing one of ethylene, propylene, and butylene glycols.
Figures 7(a)-(c) are tables of enzyme activity and water content for gel compositions containing one of ethylene, propylene and butylene glycols at Days 0, 7, 14, 48 and 56.

### DETAILED DESCRIPTION OF THE PREFERRED EMBODIMENTS

For illustrative purposes, the principles of the present invention are described by referencing various exemplary embodiments thereof. Although certain embodiments of the invention are specifically described herein, one of ordinary skill in the art will readily recognize that the same principles are equally applicable to, and can be employed in other compositions and methods. Before explaining the disclosed embodiments of the present invention in detail, it is to be understood that the invention is not limited in its application to the details of any particular embodiment shown. The terminology used herein is for the purpose of description and not of limitation. Further, although certain methods are described with reference to certain steps that are presented herein in certain order, in many instances, these steps may be performed in any order as may be appreciated by one skilled in the art, and the methods are not limited to the particular arrangement of steps disclosed herein.

The invention is directed to enzyme-containing gel compositions and methods for treating wounds, skin care, treating diseases, treating viral infections, sterilization and assisting transplants using enzyme-containing gel composition. The enzyme-containing gel composition can be tailored to provide a controlled enzymatic release and/or increased enzymatic activity level. The composition comprises at least one enzyme, at least one gelling or structuring agent and at least one diluent component. Conventional accelerators and/or preservatives and may also be optionally included.

Any enzyme, preferably at least one proteolytic enzyme, and more preferably at least one krill derived enzyme, may be encapsulated in the gel composition. The gel composition is compatible with all enzymes and uniformly functions to control enzymatic release and/or increase enzymatic activity. In general, a suitable enzymatic concentration may range from about 0.01 U/ml to about 10 U/ml and, more preferably, may range from about 0.1 U/ml to about 6 U/ml. One measure of a unit of activity is that one unit (U) of activity causes the liberation of 1 µmole of tyrosine from a tyrosine standard, per minute at 35°C. Other conventional measures of units of activity for enzymes may also be employed. The concentration of enzymes in the composition may depend on the enzyme bioavailability required of the gel composition. The enzyme composition may be used in an amount corresponding to the desired activity level of the gel composition for the particular application. The desired activity level may optionally be determined over a selected time period, depending upon the particular application of the gel composition.

Suitable krill enzymes are described in, for example, U.S. Patent nos. 6,524,814 and 5,958,406, the disclosures of which are hereby incorporated by reference for a description of suitable krill enzymes. Krill enzymes may also be derived from Antarctic krill using standard separation and desorption method, or Krillase^{®} may be used as a krill enzyme source. In general, a suitable krill enzymatic concentration may range from about 0.01 U/ml to about 10 U/ml and, more preferably, may range from about 0.1 U/ml to about 6 U/ml, where one unit (U) of activity causes the liberation of 1 µmole of tyrosine from a tyrosine standard, per minute at 35°C. The concentration of krill enzymes in the composition may depend on the enzyme bioavailability required of the gel composition. The krill enzyme composition may be used in an amount corresponding to the desired activity level of the gel composition for the particular application. The desired activity level may optionally be determined over a selected time period, depending upon the particular application of the gel composition.

The enzyme-containing gel composition comprises a gelling or structuring agent, which may include any pharmaceutically acceptable structuring or gelling agent, preferably glyceryl polymethacrylate, glyceryl acrylate and/or an acrylic acid copolymer.

The diluent component may include one or more polyols, such as, for example, propylene glycol, ethylene glycol, butylene glycol, glycerol and mixtures thereof. The polyol component of the diluent functions, at least in part, to control the release of activity of the enzymatic compound. In the examples below, it is shown that a glycol-containing gel having up to 5% by weight of water by weight in the diluent component demonstrates a controlled release of enzymatic activity.

The diluent component may contain up to 5% by weight of water. More preferably, diluents for the gel compositions contain up to 3% by weight of water, and, most preferably, up to 2% by weight of water. The maximum water content of a particular diluent component will depend on the particular diluent employed, as well as the desired enzymatic activity level and/or desired duration of enzymatic activity. Enzymatic activity may be measured indirectly by determining degradation of substrate (gelatin) over time through profilometry.

Due to the effect provided by the polyol-containing diluent, it is not necessary to completely dry the enzyme during enzymatic preparation. For example, when working with krill enzymes, the conventional freeze-drying method may be replaced with a technique of reducing the enzyme water content and then diluting it with a polyol. The attenuated activity upon rehydration is both surprising and significant. Over time the rehydrated krill enzyme compositions demonstrate an effective level of enzymatic activity and the level of activity is, to some extent dependent on one or both of the enzyme concentration and the selection of the polyol. This allows the formulator to tailor the enzyme compositions to the specific needs of particular applications.

To increase potency and enzymatic activity, suitable conventional accelerators, such as metal ions such as zinc or other additives, may be included in the enzymatic composition. For example, the addition of urea results in a significant increase of enzymatic activity, ranging between 20-30%.

Optionally, the composition may include at least one preservative such as a standard pharmaceutically acceptable preservative. Suitable preservatives include methyl paraben, ethyl paraben, propyl paraben and/or polyhexamethylenebiguanide.

The incorporation of polyol diluents, such as glycols or glycerols, functions to control enzymatic release. The controlled release mechanism of the polyol-containing diluent can be employed to deliver a substantially constant enzymatic activity over an extended period of time, provided that the water content of the diluent is maintained at a suitably low level. While not wishing to be bound by a particular theory, experiments suggest that glycol and/or glycerol associates with active sites of the enzyme rendering these active sites inactive either by chemical complexation or by causing reversible conformational changes in the protein that inactivates the protease. When placed in water, glycol and/or glycerol appear to slowly disassociate from the enzyme or at least partially disassociate from the enzyme, which provides additional enzyme activity available over time, thereby resulting in attenuated enzyme activity.

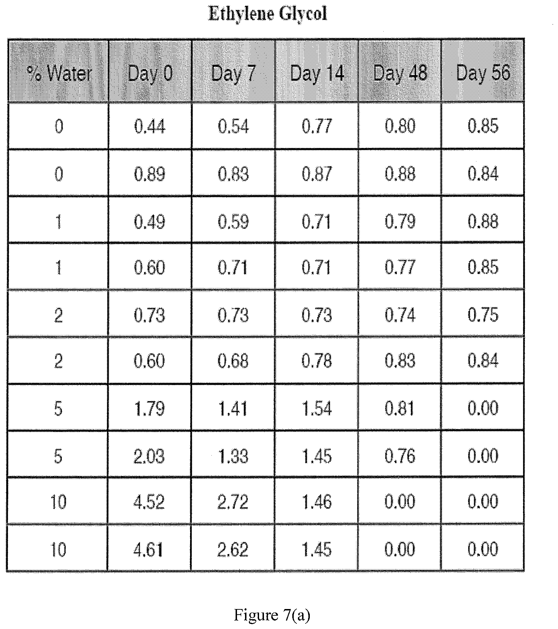

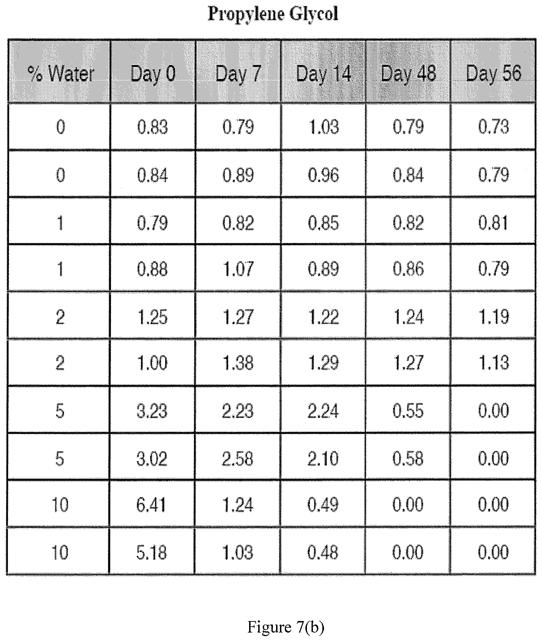

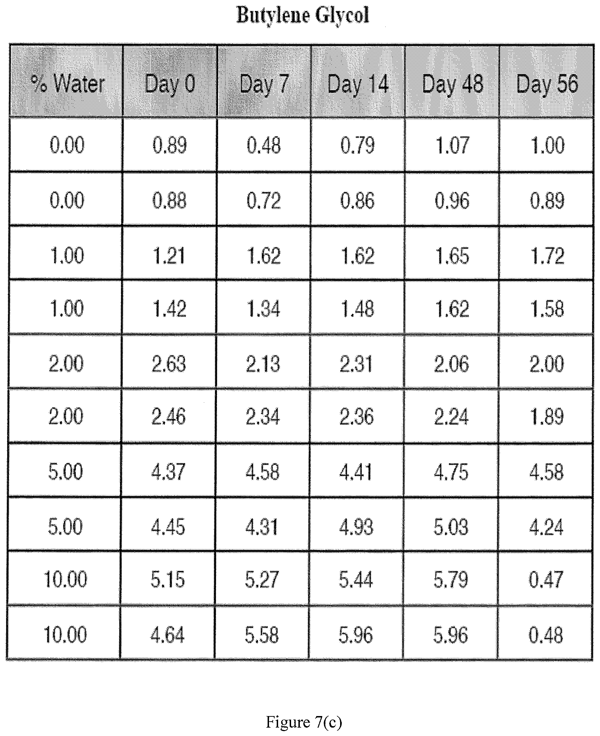

In addition to sustaining enzymatic stability and controlling activity, the controlled release mechanism may be used to increase the overall enzyme activity level, when integrated over time, thereby maximizing enzymatic efficiency. The type of polyol incorporated in the enzymatic composition may be used to control and increase the enzymatic activity level. Experiments show that different glycols may influence release and duration of enzymatic activity to different degrees. For example, Figures 7(a)-7(c) show that enzymatic activity of a composition including ethylene glycol is different than that of propylene glycol, which, in turn, is different than that of butylene glycol. By changing the enzyme-polyol composition, it is possible to achieve different degrees and/or durations of enzymatic release. It is therefore possible to customize krill enzyme gel compositions by carefully selecting the polyol component of the diluent to be incorporated in the enzymatic composition.

Preferred embodiments of the gel composition, without the enzyme, include about 1 to about 50 weight percent gelling agent, and about 50 to about 99 percent by weight diluent. More preferably, embodiments of the gel composition, without the enzyme, typically contain about 20-30 weight percent of a gelling agent and about 70 to about 80 weight percent diluent.

Optionally, the enzyme-containing gel is composed of about 0.02 to about 0.4 weight percent of preservatives. The enzymatic composition is not limited to the above compounds. Formulations may include additional elements such as mineral oil, almond oil, emulsifiers, and agents capable of gelling a glycol and/or glycerol compound, accelerators and/or any other additives.

The enzyme-containing gel compositions in accordance with the present invention are characterized by their ability to control enzymatic release and thereby sustain enzymatic activity over time. Thus, in one embodiment, suitable gel compositions maintain at least 65% of the initial enzymatic activity after 56 days. More preferably, suitable gel compositions maintain at least 75% of the initial enzymatic activity after 56 days. Most preferably, suitable gel compositions maintain at least 90% of the initial enzymatic activity after 56 days. The sustained enzymatic activity and stability may also be observed in gel compositions incorporating other proteolytic enzymes or any other enzyme.

In another embodiment, the novel enzyme-containing gel compositions in accordance with the present invention are characterized by their ability to control enzymatic release and thereby achieve a desired level of enzyme activity during a treatment or use period. For example, in Figures 7(a)-7(c) it can be seen that krill enzyme-containing gels made with 6U of activity of krill enzymes can be customized to deliver anywhere from about 0.5-6.0 units of activity at a particular time, depending upon the particular diluent composition employed. This permits simplified tailoring of the activity of gel compositions since standard krill-enzyme sources can be diluted with different diluents to provide different levels of enzyme activity, as desired, without the need to measure or determine units of activity of the enzyme source added to the composition. Thus, the diluent can be selected, for example, to deliver from about 10% to about 90% of the enzyme activity of the source material, alternatively, from about 25% to about 85% of the enzyme activity of the source material, and, optionally, about 50% to about 80% of the enzyme activity of the source material. An advantage of this aspect of the invention, demonstrated in Figures 7(a)-7(c) is that the delivered enzyme activity typically remains relatively constant over time thereby reducing variability of the enzyme treatment. The constant enzymatic activity and tailored enzymatic activity levels may also be observed in gel compositions incorporating other proteolytic enzymes or any other enzyme.

When the novel enzyme-containing gel composition incorporates at least one krill derived enzyme, a rapid and efficient debridement of necroses and/or other beneficial enzymatic effects may be achieved. Krill derived enzymatic gel compositions may be incorporated in dressings, gels, foams or other therapeutic delivery mechanisms. The enzymatic gel composition may also be delivered to a treatment site by means of injection, ingestion, or transdermal application, such as transmucosal or topical application. When intradermal penetration is required, accelerators, intradermal patches or other penetration enhancers such as iontophoresis, may be used.

In one embodiment, the krill enzyme-containing gel composition is for controlled release of krill enzymes to be administered at least once a day. The gel may also be applied every other day, especially during wound healing. As a result of the controlled enzymatic release mechanism, krill enzyme-containing gels have a number of applications. By incorporating krill enzymes in a controlled-release gel, tissue debridement may be enhanced. Krill enzyme-containing gel compositions in accordance with the present invention are efficient in removal of devitalized tissue in different types of necrotic ulcers (venous/arterial, traumatic/surgical), decubitus, burns, plastic surgery and other deep tissue lesions as abscesses, fistulas, pleuritis, empyema, hematothorax, osteomyelitis, otitis, sinusitis, topical ulcers (lepra, leishmaniasis).

It is envisaged that the krill enzyme-containing gel compositions of the present invention may also be used to treat skin disorders, wounds, certain diseases and viral infections. Treatment of skin disorders and improving skin appearance may include treatment of dermatological disorders like acne or psoriasis. Treatment of wounds, diseases and viral infections may include treating fungal infections, bacterial infections, parasitic infections, hemorrhoids, corneal scarring, dental plaque, gingivitis, periodontitis, fungal stomatitis, aphte or canker ulcerations, oral mucositis, leukoplakia, cheilitis, cystic fibrosis, blood clots, immune disorders, autoimmune diseases, cancer, herpes, and other viral infections such as herpes zoster or varicella. As topical microbiocide it could prevent rectal and vaginal transmission of HIV and also protect against other sexually transmitted disorders such as chlamydia or gonorrhea. Additionally, the composition may also be useful as a cleansing agent to sterilize medical instruments, such as endoscopes, contact lenses and catheters, and as an adjunct to diverse transplantation solutions for organs, tissues or cells prior to implantation.

A krill enzyme gel is also advantageous in its ability to be topically applied to the skin. Unlike abrasive scrubs or other mechanical/chemical methods, enzymatic exfoliation is an acceptable alternative for those patients seeking softer, smoother skin. This natural, effective and non-irritating methodology is beneficial for skin rejuvenation, dry skin, acne, hyperpigmentation and scar reduction. Krill enzyme-containing gel compositions of the invention may be used in a variety of topical preparations (creams, cleansers, masks) to gently exfoliate the skin leaving a soft smooth surface.

One issue to be considered in connection with enzymatic treatment or peeling is the bioavailability of the enzyme. A suitable vehicle must allow sufficient enzyme release together with sufficient penetration of the enzyme into the skin. It is also important that the enzymatic activity remains predictable and that the enzymatic release is controlled. In addition, biodegradability, toxicity or immunogenicity of such a vehicle should not cause problems.

The instant polyol achieves a physiologically compatible formulation assuring desirable enzyme release properties into the skin. The combination of krill enzymes with such a vehicle improves their stability and performance providing a safe and effective system for topical delivery.

### Examples 1-2

Exemplary enzyme compositions include:
Enzymatic Composition 1
   5% glyceryl polymethacrylate
   94% propylene glycol
   0.13% methyl paraben
   0.04% propyl paraben
   krill-derived enzymes corresponding to the desired activity level
Enzymatic Composition 2
   5% glyceryl acrylate/acrylic acid copolymer
   76% Propylene glycol
   15% glycerol
   0.13% methyl paraben
   0.04% propyl paraben
   krill-derived enzymes corresponding to the desired activity level

### Methods of Production

In order to maintain the natural composition of the krill enzymes during extraction and purification, the raw material is defatted, extracted using a two-phase equilibrium separation, ultrafiltrated and size excluded using gel chromatography. The resultant sterile, lyophilized powder is then reconstituted.

Exemplary methods for producing the enzymatic composition include:

### Method of Krillase^{®} Extraction and Preparation

Antarctic krill (*Euphausia Superba*) extracts were prepared by first freezing Antarctic krill at -30°C. The frozen krill was then thawed, homogenized and defatted. The mixture was centrifuged (5,000 x g for 30 min at 4°C). The supernatant, a crude extract, was concentrated in an Amicon^{™} hollow fiber concentrator equipped with a UM-10 filter and filtered through a 0.45µm Millipore membrane filter. The concentrate extract was then applied to a Sephadex G-75 column, equilibrated with a 0.05 M Tris-HCl solution having a pH of 7.5. Throughout this process, the absorbance was continuously monitored at 280 nm and fractions were collected and analyzed for trypsin activity such as hydrolysis of TAME and p-toluene-sulfonyl-L-arginine methyl ester.

The proteolytic activity was established with denatured casein as a substrate. After 20 minutes, the reaction of 0.1 M Tris-HCl, pH 7.5 was stopped by adding 5% of trichloroacetic acid. After high-speed centrifugation the sediment was removed from the mixture and the resultant supernatant was measured with a Folin-Ciocalteau phenol reagent and a tyrosine reference point.

### Method for Manufacturing the Enzymatic Composition 1

Mixture A: 5 g glyceryl polymethacrylate were combined with 80 g propylene glycol and heated to 80°C with rapid stirring until a clear solution resulted. The mixture was then cooled to 50°C.

Mixture B: 0.13 g methyl paraben was combined with 0.04 g of propyl paraben in 14 g propylene glycol solution with rapid stirring at room temperature.

Mixture A and Mixture B were combined with rapid stirring at room temperature. The combined mixture was then cooled to 30°C with constant stirring. Freeze-dried krill enzymes were added to the anhydrous gel in an amount sufficient to result in a proteolytic activity of 6 U/g of finished gel. The gel mixture was then efficiently stirred to room temperature and allowed to stand at room temperature for at least 24 hours.

### Method for Manufacturing the Enzymatic Composition Production 2

Mixture A: 8 g of glyceryl acrylate/acrylic acid copolymer was combined with 76 g of propylene glycol and heated to 80°C with rapid stirring until a clear solution resulted. Mixture A was allowed to cool to 50°C.

Mixture B: 0.13 g methyl paraben was combined with 0.04 g propyl paraben in 15 g glycerol with rapid stirring at room temperature.

Mixture B was added to Mixture A with rapid stirring. The combined mixture was then allowed to cool to 30°C with constant stirring. Freeze-dried krill enzymes were added to the anhydrous gel in an amount sufficient to result in a proteolytic activity of 6 U/g of finished gel. The gel mixture was efficiently stirred to room temperature and allowed to stand at room temperature for at least 24 hours.

### Example 3

To prepare krill enzymes from Antarctic krill, the frozen krill was homogenized in water or buffer, preferably containing an antimicrobial agent. The supernate, which may be diluted if appropriate, may then fractionated by ion exchange chromatography (preferably anion exchange chromatography), gel filtration, chromatofocusing chromatography, or other traditional separation processes. Preferably, however, some part of the separation process will include affinity chromatography using a matrix having attached molecules of a trypsin inhibitor, such as soybean trypsin inhibitor. The krill-derived enzymes were then desorbed from such a matrix by applying conditions that will destabilize the interaction between the enzyme and the inhibitor. Such conditions include high salt content, low pH or the presence of denaturants such as urea. To add another selective process, the destabilizing condition may be applied to the matrix incrementally, as in a gradient. When affinity chromatography is used, it is preferably followed with chromatography using a matrix having attached molecules of the multifunctional enzyme used in the invention. This enzyme affinity step serves to remove molecules of trypsin inhibitor that leach off the first affinity matrix.

Protease activity was then determined by incubating an enzyme preparation with casein (concentration: 1% w/v) at 30 °C for 20 hours and measuring the release of amino acids or peptides (which can be measured by the increase in colorometrically determinable amino groups). Casein Units are defined in Biochem. J., 173: 291-298, 1978 (using azocasein as the substrate). Alternatively, tryptic protease activity were measured against tyrosine-arginine-methyl-ester ("TAME"). Or, tryptic activity can be measured using Benzoyl-Val-Gly-Arg-p-NO.sub.2 -anilide as the substrate. Chymotryptic activity can be measured using Succinyl-Ala-Ala-Pro-Phe-p-NO.sub.2 -anilide as the substrate. Elastase activity can be measured using Boc-Ala-Ala-Pro-Ala-p-NO.sub.2 -anilide as the substrate.

### Example 4

Krillase^{®} may be prepared from frozen -30°C Antarctic krill (Euphausia Superba) extracts. The frozen krill raw material is thawed, homogenized and defatted. The mixture is centrifuged (5,000 x g for 30 min at 4°C); The supernatant, a crude extract, is concentrated in an Amicon^{™} hollow fiber concentrator equipped with a UM-10 filter and filtered through a 0.45µm Millipore membrane filter. The concentrate extract is then applied to a Sephadex G-75 column, equilibrated with a 0.05 M Tris-HCl solution having a pH of 7.5. Throughout this process, the absorbance is continuously monitored at 280 nm and fractions are collected and analyzed for trypsin activity, by, for example, determining hydrolysis of TAME and p-toluene-sulfonyl-L-arginine methyl ester.

### Example 5

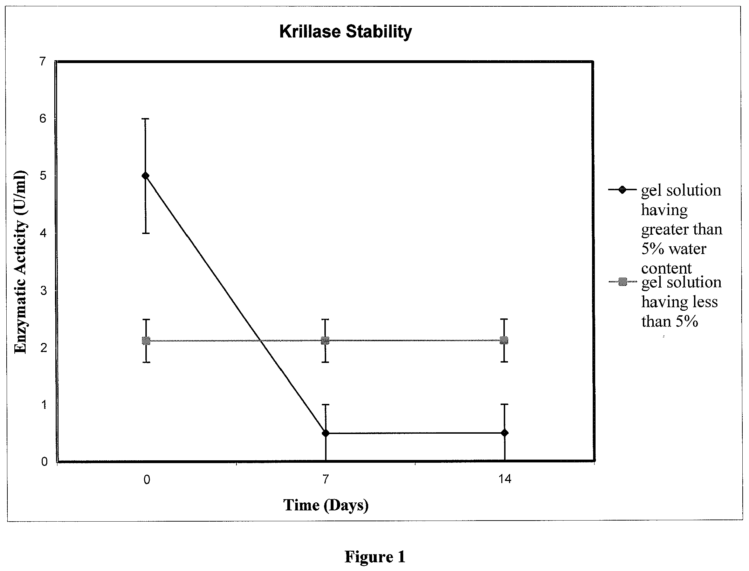

The activity levels of Krillase^{®} incorporated in various gels in combination with glycol-containing diluents were studied. The diluents included ethylene glycol, propylene glycol and butylene glycol containing 0%, 1%, 2%, 5%, and 10% by weight of water. The gels were maintained in a 35°C chamber over a period of 2 weeks during which enzymatic activity was measured at regular intervals. Figure 1 shows that gels having up to 5% by weight of water consistently maintained activity levels of 1.75-2.5 U/ml in comparison to declining activity levels measured in gels having greater than 5% by weight of water.

### Example 6

Enzymatic stability was studied and found to be improved in glycol gels containing up to 5% by weight water. This experiment further established the viability of a glycol delivery vehicle for Krillase^{®} and demonstrated that Krillase^{®} does not conform to the generally expected protease properties. All percentages in this example are given as percentages by weight.

In this experiment, a set of 6 U/ml Krillase^{®} samples, containing 60 activity units (AU)/10 mL of Krillase^{®} were dissolved in 15 different diluents and maintained in a chamber at 35±0.5°C. The 15 diluents included ethylene glycol, propylene glycol and butylene glycol, separately containing 0%, 1%, 2%, 5%, and 10% by weight of water. Enzymatic activity assays were regularly measured over a period of 8 weeks, during which time it was established that enzymatic activity was related to the amount of water in the diluent.

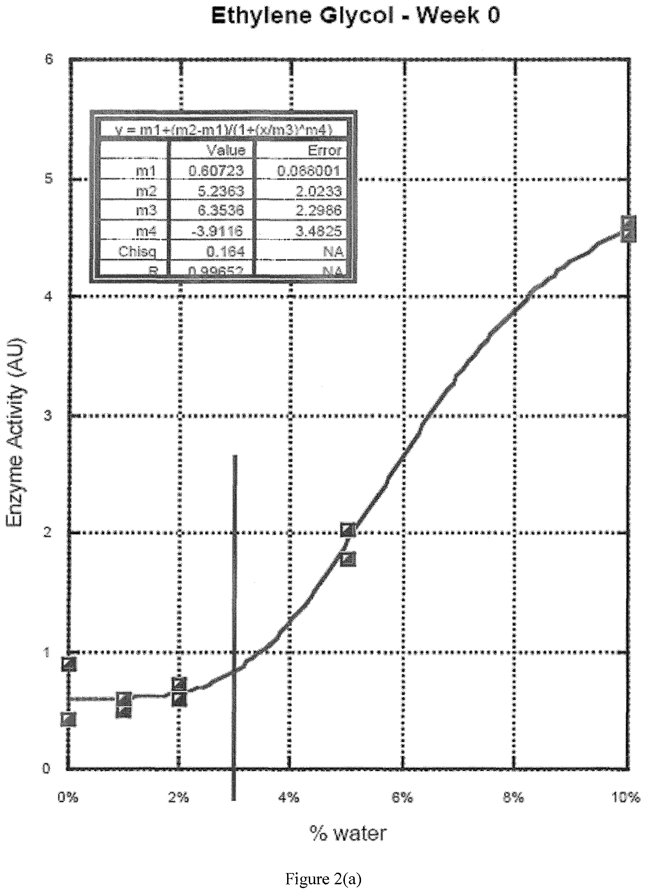

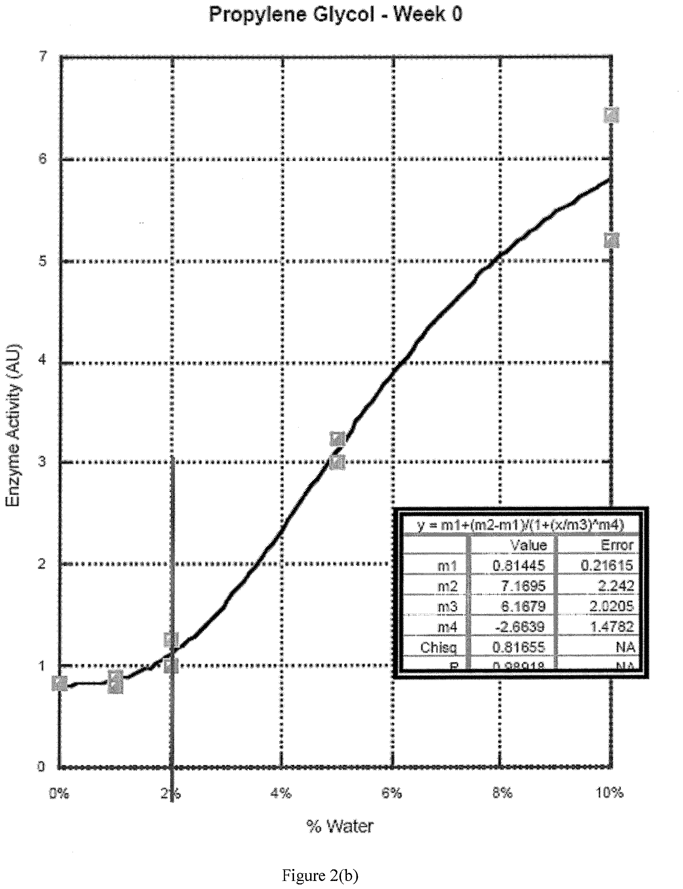

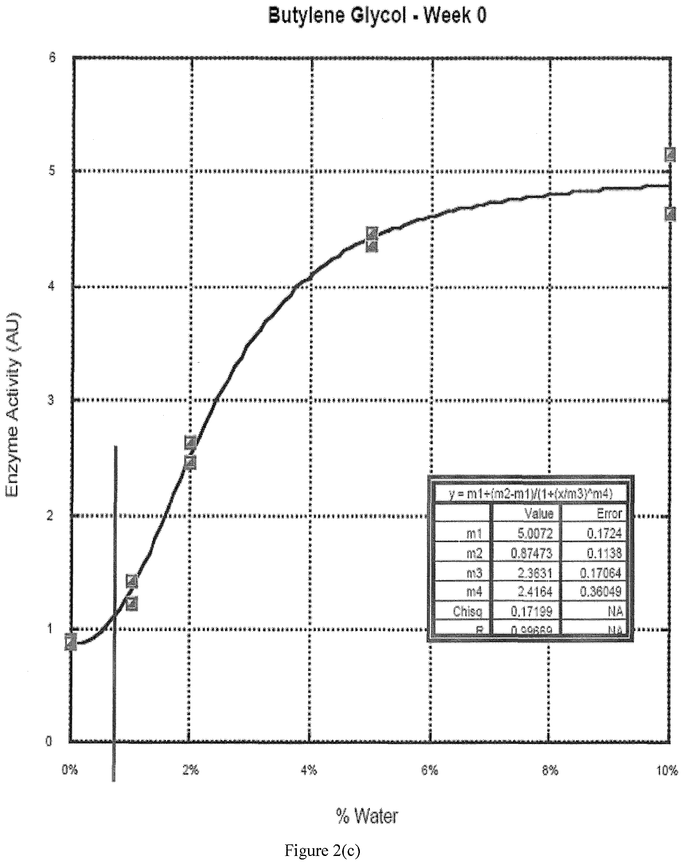

The Krillase^{®} solutions were prepared on Day 0 and subsequently placed in a stability chamber at 35°C. On Day 0, the solutions were tested for enzyme activity by profilometry using casein as a protein substrate. An aliquot of the Krillase^{®} glycol solution was diluted with an aqueous 0.1M TRIS buffer solution. Although it was expected that dilution would release the Krillase^{®} from the glycol solution, Krillase^{®} activity, however, remained substantially proportional to the water content of the original solution. Figs. 2(a)-2(c) shows that all of the test solutions demonstrated complete enzyme activation/release when diluted to 10% water content. This suggests that the glycols may become tightly bound to Krillase^{®} and form a complex that is not easily solvated nor disassociated simply by dilution with water.

The results suggest that Krillase^{®} release is related to the nature of the glycol. The non-linear relationship between AU/ml and water content in Figs. 2(a)-2(c) demonstrates that enzyme activity is not simply a function of the water concentration in the solution. Further, these figures show that the data curves are sigmoidally dependent on two independent variables, suggesting perhaps, that at low water levels, a unique complex is formed between glycol and Krillase^{®} that is more stable and less soluble than a complex formed with higher water content. The initial flat portion of each curve in Figs. 2(a)-2(c), which represents the more stable complex, shows no relationship between water concentration and enzymatic activity.

Figs. 2(a)-2(c) also show that the "break point", the point at which proteolytic activity begins to increase rapidly in relation to water content, varies inversely with the molecular weight of the glycol. The lower the molecular weight of the glycol, the higher the water concentration necessary to prevent formation of the more stable complex. Enzyme activity may then be maximized by manipulation of the molecular weight of the glycol. Table 1 shows the break points at Day 0. Each of the glycols reduced the activity or attenuated the enzymatic activity to a degree inversely proportional to their carbon chain length.

**Table 1: Break Points for the Various Glycol Solutions**

| **Glycol** | **Break Points** | | | | |
|---|---|---|---|---|---|
| | **Day 0** | **Day 7** | **Day 14** | **Day 48** | **Day 56** |
| Ethylene glycol | 3% H₂O | 3% H₂O | 2.75% H₂O | 3% H₂O | 2% H₂O |
| propylene glycol | 2% H₂O | 1% H₂O | 1.25% H₂O | 1.25% H₂O | 1% H₂O |
| Butylene glycol | 0.75% H₂O | 0.5% H₂O | 0.5% H₂O | No break point | No break point |

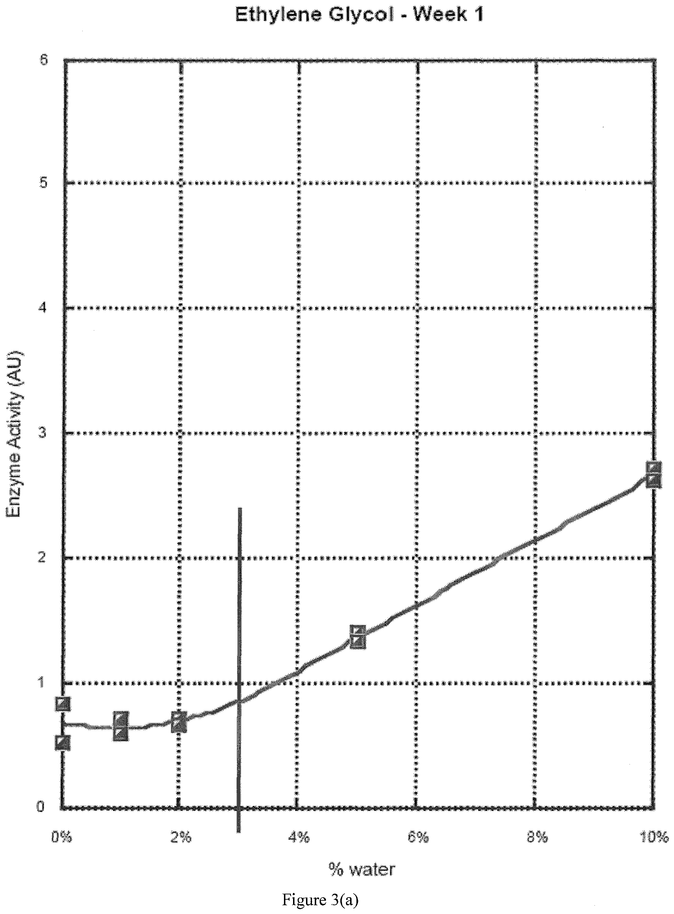

On Day 7, all of the solutions were again tested for proteolytic activity and subsequently returned to the stability chamber. Figs. 3(a)-3(b) show the Day 7 results, with both the ethylene glycol and propylene glycol solutions containing a 5% or 10% water content, lost considerable enzyme activity, in comparison to Day 0. The enzyme activity of the butylene glycol solutions remained relatively stable.

Although the activity data for ethylene glycol and propylene glycol solutions no longer exhibited a sigmoidal relationship to water content, the flat initial portion of the activity curves still suggests the formation of a tight glycol-krillase complex. The observed break points for these tight complexes are listed in Table 1. While not wishing to be bound by theory, the loss of enzymatic activity may be caused by self destruction of krill enzyme in the glycol solution. Therefore, it is hypothesized that solutions losing significant proteolytic activity over time do not appear to contain glycol-stabilized enzyme.

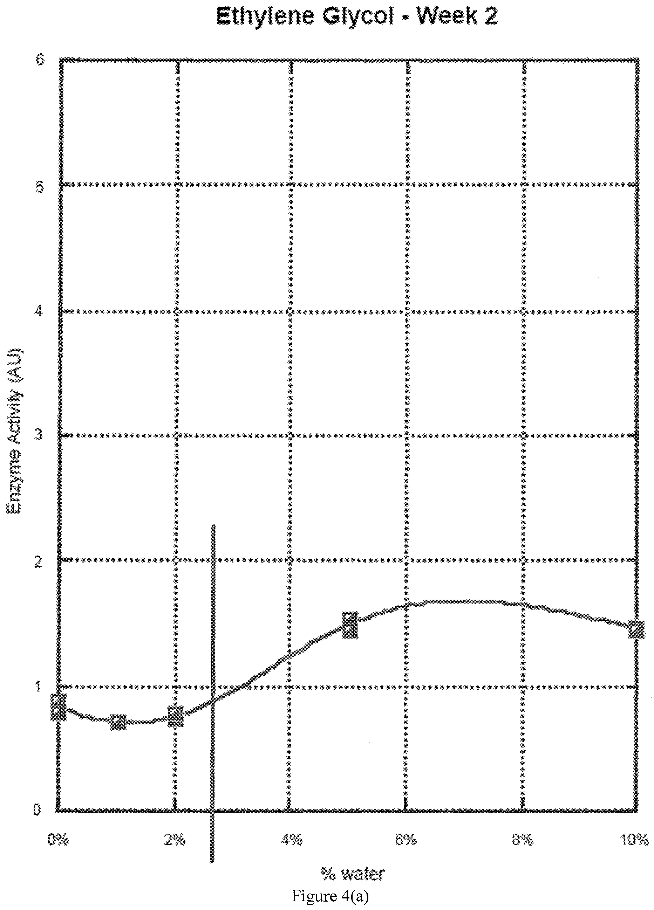

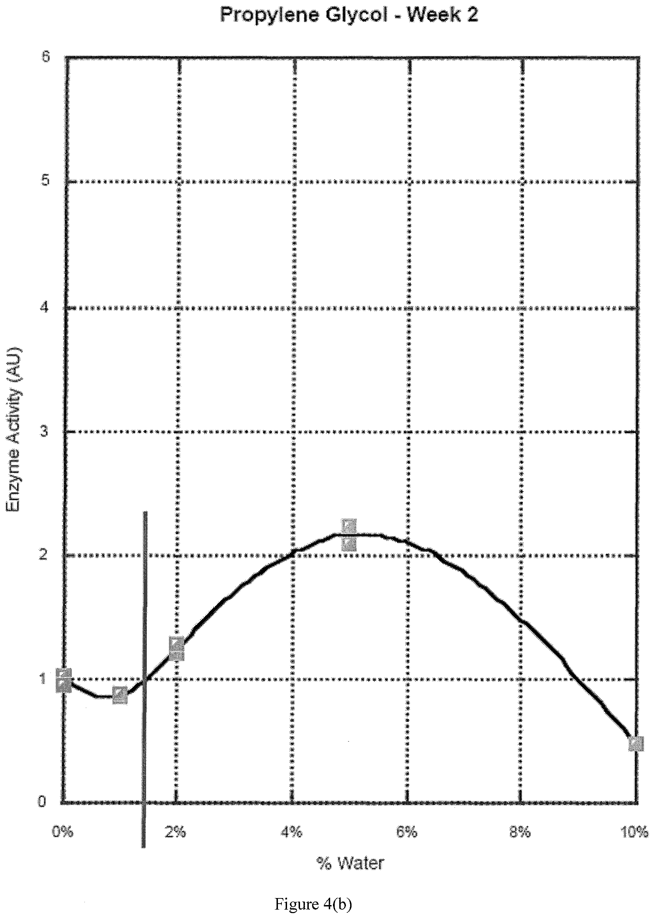

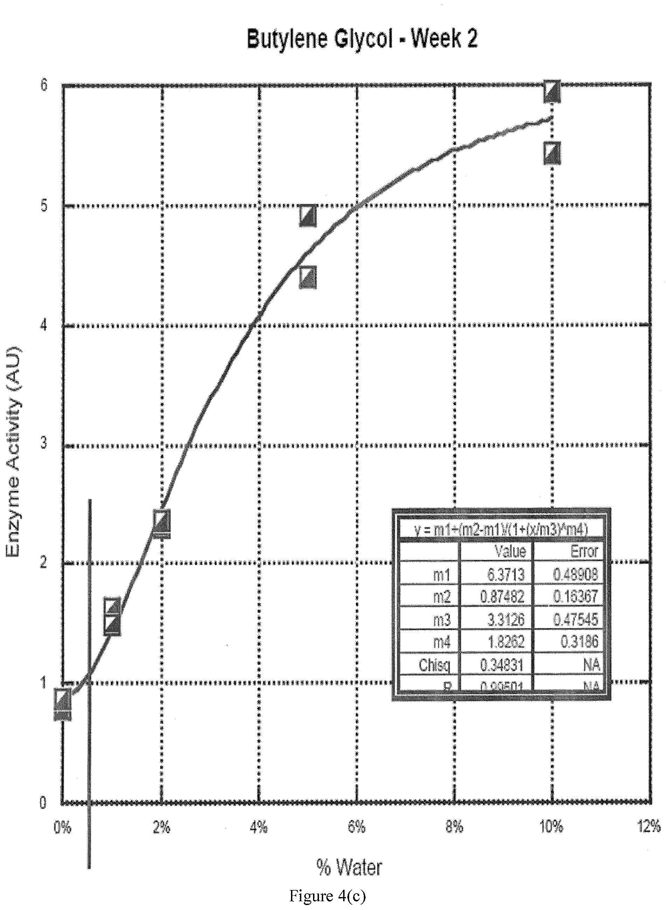

After 14 days, the solutions were tested for proteolytic activity and returned to the stability chamber. Figs. 4(a)-4(c) summarize the results. Ethylene glycol and propylene glycol solutions lost all enzyme activity when the solution was diluted to a 10% water content; the remaining ethylene glycol and propylene glycol solutions where essentially unchanged from Day 7. The butylene glycol solutions remained relatively stable. Table 1 shows the break points for these complexes.

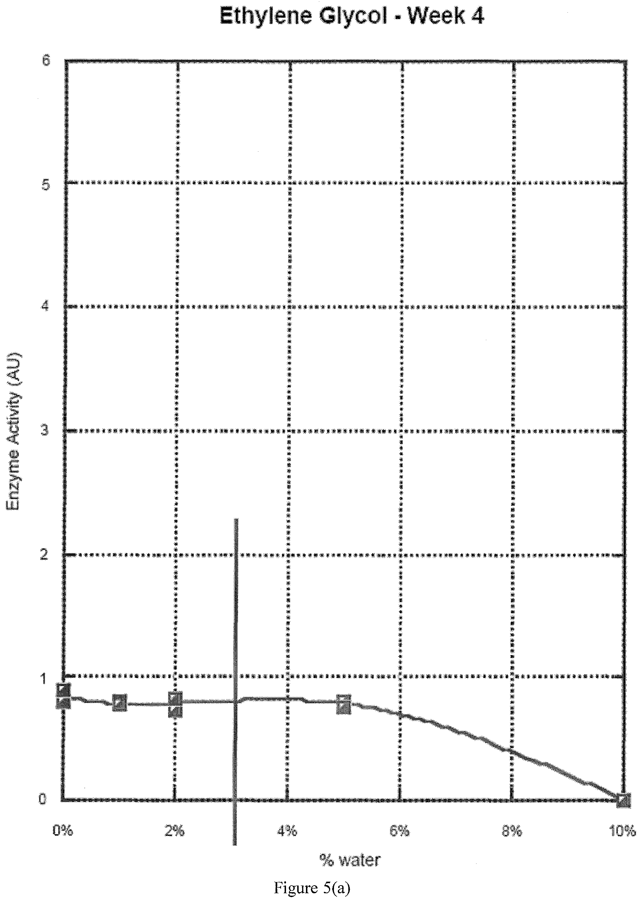

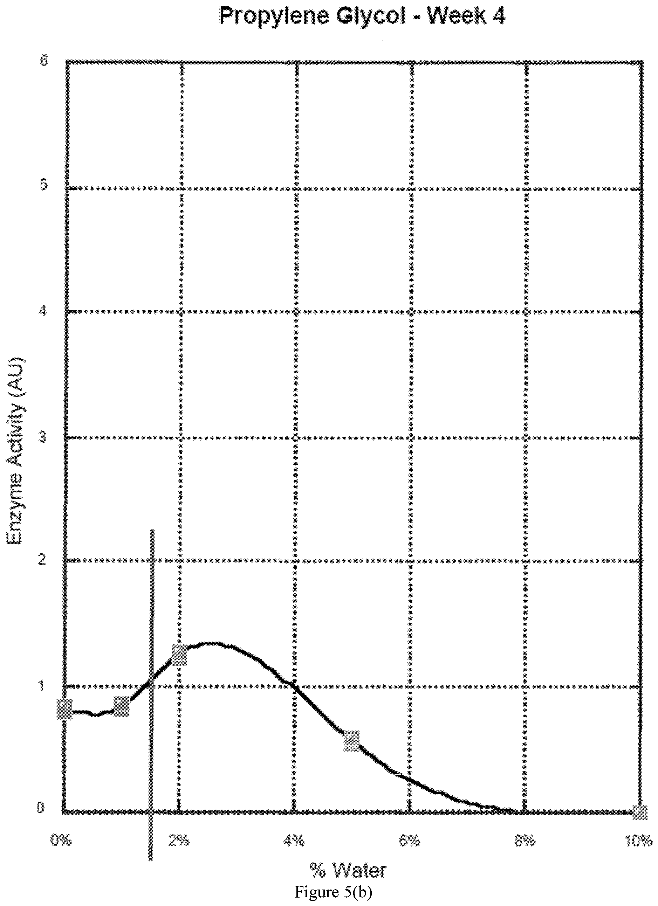

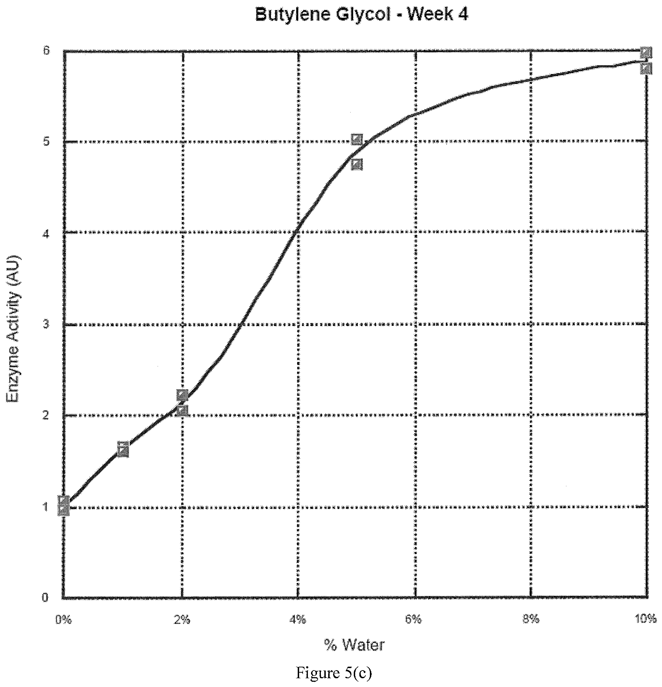

After 28 days, the solutions were tested for proteolytic activity and returned to the stability chamber. Fig. 5(a)-5(c) show that ethylene and propylene glycol solutions lost all enzyme activity when diluted to a 5% or 10% water content while butylene glycol solutions remained relatively stable. Table 1 shows the break points and Fig. 10(c) shows that the degradation of the butylene glycol complex.

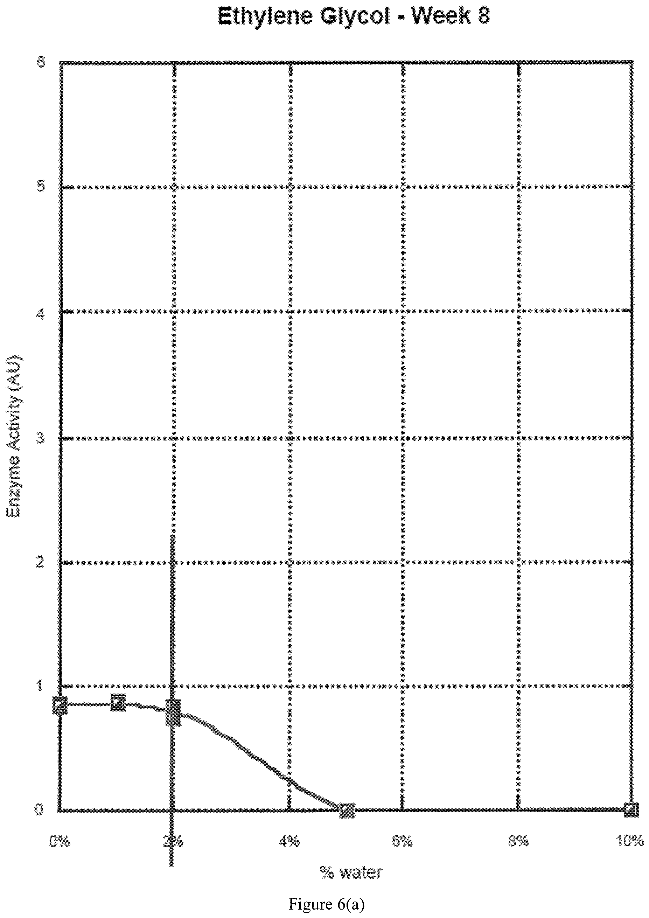

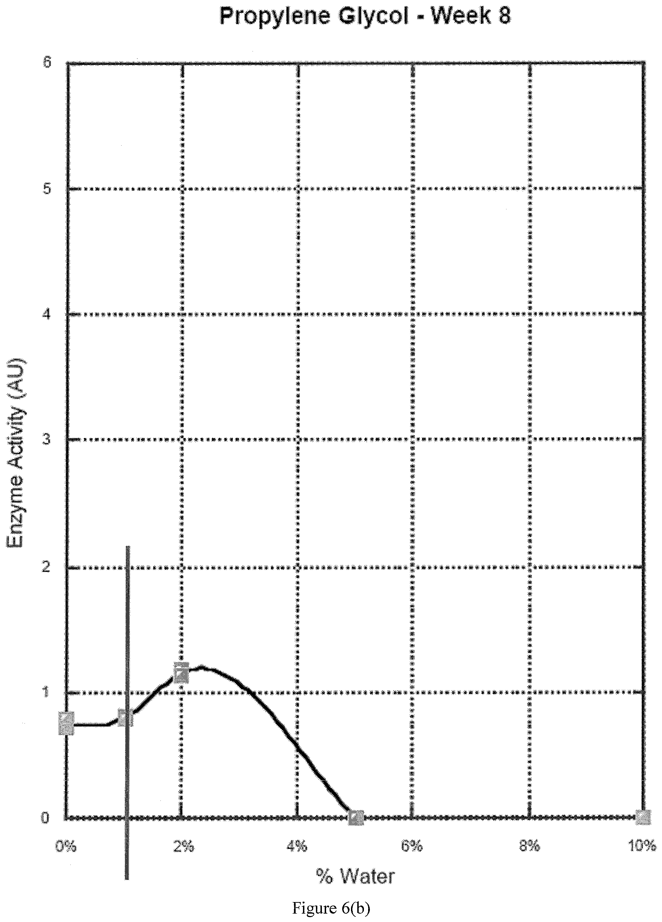

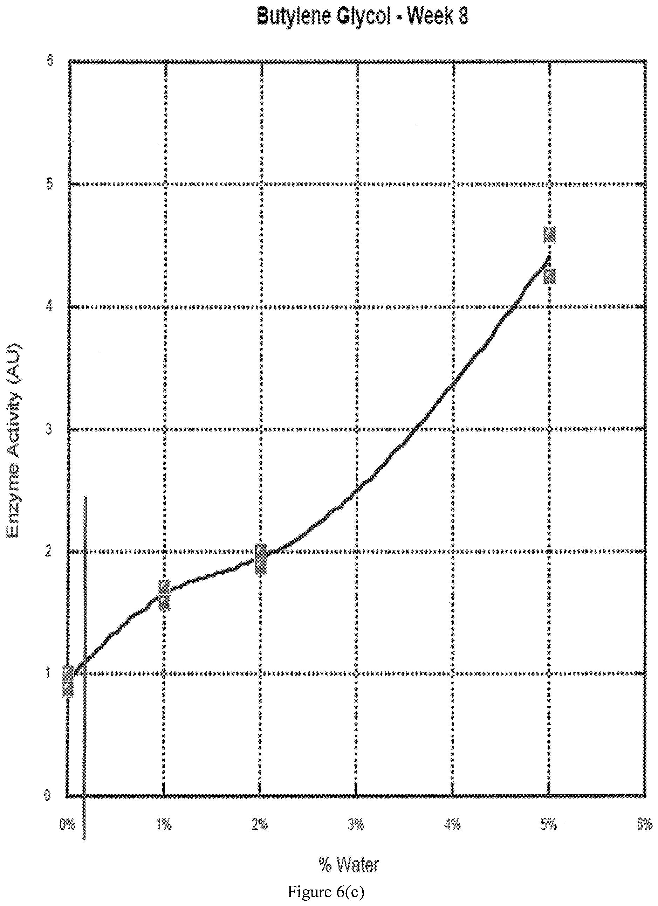

After 56 days, the solutions were tested for proteolytic activity and returned to the stability chamber. Figs. 6(a)-6(c) shows that except for what might be complexes, all of the ethylene and propylene glycol solutions lost all enzyme activity. Further ethylene and propylene glycol solutions, having a water content of 2% or more, lost all enzyme activity; butylene glycol solutions having a water content of about 10%, also lost all enzyme activity. Butylene glycol solutions, having a water content of 5%, also lost enzyme activity. Moreover, all of the butylene glycol solutions with a water content of 2% or less remained relatively stable. The corresponding break points are shown in Table 1.

The study demonstrates that low molecular weight glycols do not significantly stabilize Krillase^{®} at water concentrations above 2%, that Krillase^{®} appears to form glycol-enzyme complexes in solutions containing 2% water or less, that the ease of formation and stability of glycol-enzyme complexes appears to be inversely proportional to the molecular weight of the glycols, and that the maximum proteolytic activity and the duration of activity of Krillase^{®} formulations may be adjusted by varying the content and type of glycol ingredients. Because of these unusual properties of Krillase^{®} formulated with various glycols, it may be possible to control the release of krill-derived enzymes with a wide range of liquid phases and mixtures.

### Example 7 and Comparative Examples A-B

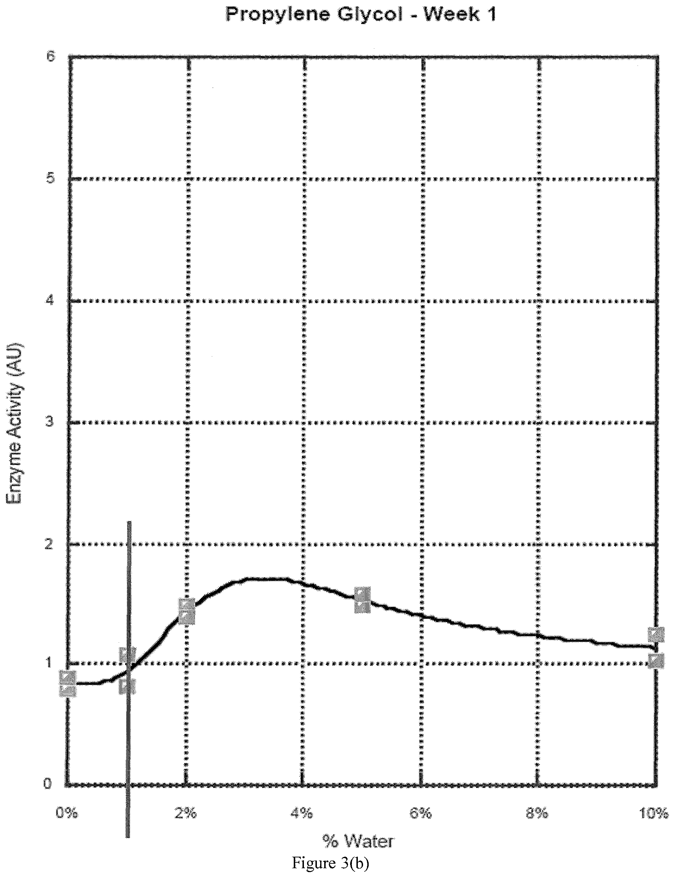

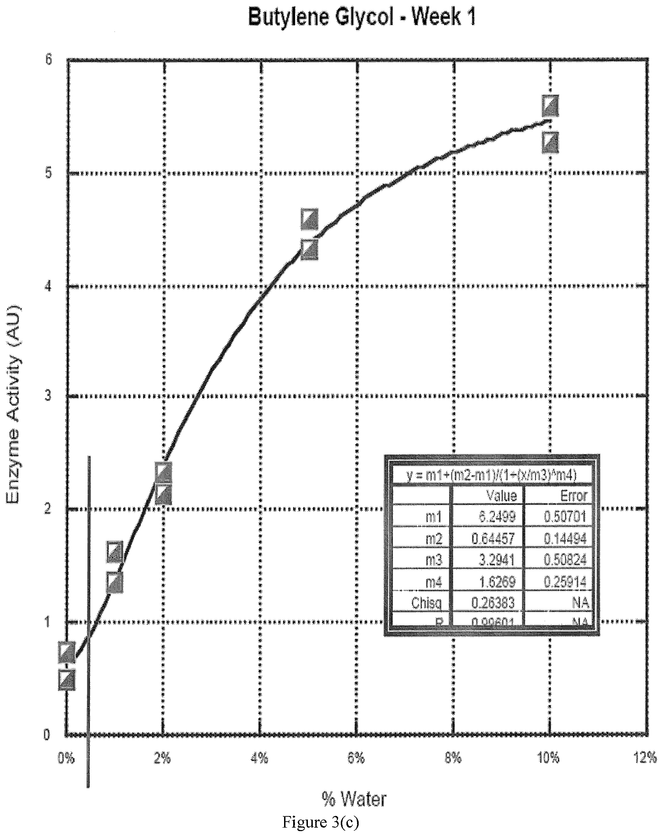

Experiments show that glycols elevate the bioavailablity of krill enzyme. As shown in Figure 3, a 3 U/ml concentration of Krillase^{®} was incorporated in a saline solution (Comparative Example A), a hydrogel (Comparative Example B), and a gel formulated according to this invention (Example 5) with a standardized thin gelatine film substrate. Enzymatic activity was quantified by gradual enzymatic decomposition of gelatine using advanced profilometric technology, which uses computer-assisted optical surface measuring systems to measure 2D and 3D profiles based on digital micro-mirror projections. Figure 3 illustrates that although the fastest enzyme release was initially observed in the saline solution and hydrogel-containing compositions, enzyme activity was exhausted within a few hours. The glycol gel of Example 5, in comparison, continued to be active for at least 10 hours. These findings further substantiate the excellent controlled and sustained release properties of the gel of the present invention. Therefore by incorporating Krillase^{®} in a glycol and/or glycerol gel, the therapeutic dosage of Krillase^{®} may be substantially decreased while maintaining a desirable level of efficacy over a period of time.

### Examples 8-10 and Comparative Examples C-D

Example 21 of US Patent no. 6,030,612, (Comparative Example C), employed a krill enzyme-containing saline solution for treating dental infections. 22 patients with acute or chronic gum infections/inflammations were treated 3 times a day with a dose of 5 casein U/ml (5 U/ml of krill enzyme solution). Pain diminished after 20 minutes to 12 hours and infections and inflammations were healed within 4 days. Pursuant to this treatment plan, a total of 180U of solution was necessary for treatment (3 ml of solution at 45U/ml/day x 4 days).

In a separate experiment, Comparative Example D, analogous results were obtained when treating a healthy 40-year old female patient with recurrent aphthous stomatitis. A 1 cm² gauze saturated with a 6U/ml krill enzyme-containing saline solution was applied directly on the lesion for 10 minutes. For the first 5 minutes, the cheek was positioned and the mouth held open to reduce saliva contact and rapid dilution of the active component. This procedure was repeated 3 times daily for 7 days until healed. Treatment with krill enzymes resulted in a faster 7 day healing period in comparison to the 9 day placebo treatment of a similar lesion in the same patient. Further, pain and discomfort, measured on a VAS-scale, was dramatically reduced within a few hours upon treatment with krill enzymes and completely eliminated within three days. In this clinical study, a total of 378U of solution was necessary for treatment (3 ml/treatment at 54U/day x 7 days).

Examples 6-8 employed a Krillase^{®} glycol gel on various patients. Based on these experiments, the total amount of Krillase^{®}, when used in gel form, may be reduced at least by a factor of 10 as compared to Krillase^{®} in saline solution. The Krillase^{®} gel formulation was found to be more efficient and effective, as compared to Krillase^{®} saline formulations, in treating the same diseases, probably due to increased enzyme bioavailability at the treatment site. Moreover, the gel formulations were easy to apply and reduced patient discomfort relative to gauze applications of Krillase^{®} solutions.

In Example 6, a healthy 18-year female patient with aphthous stomatitis was treated with a gel containing 3U/g of Krillase^{®} three times a day and results comparable to Comparative Example D were obtained. The pain was reduced on a VAS-scale from 9 to 3 after 6 hours. On day 2, the pain was reduced to a VAS-score of 3, and on day 3, the VAS-score declined to 2. Within 7 days the VAS-score was reduced to 1, and the lesion was completely healed. No adverse reactions were observed. In this study, 0.5 g/treatment at 3U/day x 3 days, only a total of 9U of Krillase^{®} was necessary for treatment.

In Example 7, a healthy male patient with recurrent aphtous lesion, lasting for 2 days, on the mucosa of his lower lip was treated with Krillase^{®} gel. 0.5g of Krillase^{®} gel, composed of 6U/g of Enzymatic Composition 1, was applied 3 times a day for 8 days. The patient's VAS-score decreased from 9 to 5 in less than a day and further declined to 2 after about a day. Additionally, the intense pain associated with eating was dramatically reduced within 24 hrs of treatment. A marked clinical reduction in the redness halo around lesions and a reduction in fibrin layer, an indicator of wound healing, were also observed. By day 3, the VAS-score declined to less than 2; on day 5, the VAS-score remained at 0 until the lesion was completely healed. No adverse reactions were observed.

In Example 8, a young male patient with fresh aphtous lesions which had formed within 12 hours on the left side of the lower lip was treated with 0.5g Krillase^{®}gel, composed of 6U/g of Enzymatic Composition 1, defined above, 3 times a day for 5 days. Within 10 minutes after application, the patient described an immediate soothing and almost anesthetic effect. Additionally, within hours of treatment, the patient experienced a marked reduction in pain, declining from a VAS-score of 6 to 4 within 3 hours. On day 4, the VAS-score declined to 2. By Day 5, the VAS-score remained at 0 until completely healed; during that period, the lesion was still present on the lip but was covered with an epithelial surface. No adverse reactions were observed.

In all cases, patients reported a dramatic reduction in pain immediately after, or within the first day of applying of Krillase^{®} gel. Such pain reduction applies to both treatment at lower Krillase^{®} gel concentrations, e.g. 3U/g, and high Krillase^{®} gel concentration of e.g. 6U/g. The effect of Krillase^{®} gel was most pronounced in the patient of Example 7, who exhibited the most severe aphtous lesions. Generally, such lesions cause several days of pain. Example 6, however, demonstrated that the sustained release of a high krill enzyme concentration is capable of substantially relieving pain, especially when eating, within a day. Thus, the most effective results were obtained with Krillase^{®} gels, as compared to rinsing with Krillase solutions. Moreover, these studies also suggest that gels containing high enzyme concentrations, e.g. 6U/g of enzymatic composition 1, produce faster pain relief.

### Example 11 and Comparative Examples E-F

Examples 26-28 of U.S. Patent no. 6,030,612, Comparative Example E, employed a krill enzyme-containing saline solution for treating herpes genitalis/labialis as well as zoster. Eight patients with herpes labialis were treated with 3 ml of a 5U/ml of krill enzyme containing saline solution twice a day. After five days the patients were symptom-free and the pain was reduced between 2 hours - 2 days. A total 150U of krill enzyme containing saline solution was necessary for treatment (30U/day x 5 days).

In another experiment, Comparative Example F, a healthy 62-year old male patient with painful recurrent herpes labialis was treated twice a day with a solution of krill enzymes. A vial containing 60U/ml was reconstituted with 10ml of saline resulting in a final concentration of 6U/ml. Gauze saturated with such a solution was applied on the lesion for 10 min. Shortly after the first treatment the pain was reduced. The next day, erythema and swelling also declined, and after 3 days, the patient was symptom-free. A total of 108U of krill containing saline solution was required for treatment (6 ml/treatment at 36U/day x 3 days).

In comparison to krill saline solution, a Krillase^{®} glycol gel was found to be more effective in treating herpes. In a clinical study, Example 9, 3U/g of Krillase^{®} in glycol gel was applied 3 times a day to patients suffering from herpes. Within 3 days, the patients were without symptoms. At 0.5g gel/treatment and 4.5U/day x 3 days, a total of 13.5U of Krillase^{®} in glycol gel was necessary for treatment. This demonstrates that the total amount/treatment with Krillase^{®} when used in the form of a gel may be reduced at least by a factor 10 when compared with Krillase^{®} in saline solution. Furthermore, a sustained enzyme release may be used to optimize bioavailability of the drug at the site of infection.

Having described preferred embodiments of the invention which are intended to be illustrative and not limiting), it is noted that modifications and variations can be made by persons skilled in the art in light of the above teachings. It is therefore to be understood that changes may be made in the particular embodiments of the invention disclosed which are within the scope and spirit of the invention as outlined by the appended claims. Having thus described the invention with the details and particularity required by the patent laws, the intended scope of protection is set forth in the appended claims.

## Claims

1. An enzyme-containing gel composition comprising at least one proteolytic enzyme obtained from Antarctic krill, at least one gelling agent and at least one diluent comprising a polyol for use as a medicament, wherein the enzymatic concentration of the enzyme is from 0.01 U/ml to 10 U/ml, where one unit (U) of activity causes the liberation of 1 µmole of tyrosine from a tyrosine standard, per minute at 35°C, wherein said polyol is selected from the group consisting of propylene glycol, ethylene glycol, butylene glycol, glycerol and mixtures thereof, wherein said gelling agent is selected from the group consisting of glyceryl polymethacrylate, glyceryl acrylate and a glyceryl acrylic acid copolymer, wherein said diluent comprises up to 5% by weight water.

2. The composition of claim 1, wherein the gel composition contains from about 0.1 U/ml to about 6 U/ml of said krill enzyme.

3. The composition of any of the preceding claims, further comprising an effective amount of a compound selected from the group consisting of at least one accelerator, in particular urea, at least one metal ion and a mixture thereof to enhance the enzymatic activity of the composition.

4. The composition of any of the preceding claims, wherein said diluent comprises up to 3%, in particular up to 2%, by weight water.

5. The composition of any of the preceding claims, further comprising a preservative, in particular methyl paraben, ethyl paraben, propyl paraben, and/or polyhexamethylenebiguanide.

6. The composition of any of the preceding claims for use with a mammal as topical microbiocide, in prevention of rectal or vaginal transmission of HIV, in protection against other sexually transmitted disorders, in particular against chlamydia or gonorrhea, in tissue debridement, removal of devitalized tissue in necrotic ulcer, decubitus, burn, plastic surgery or other deep tissue lesions, in particular abscesses, fistulas, pleuritis, empyema, hematothorax, osteomyelitis, otitis, sinusitis, or topical ulcers, in particular in lepra or leishmaniasis, in scar reduction, in sterilization, or in assisting transplants, or for use in the treatment of hyperpigmentation, a wound, an infection, a necrotic wound, a skin disorder, in particular acne or psoriasis, a fungal infection, a bacterial infection, a viral infection, a parasitic infection, a hemorrhoid, corneal scarring, dental plaque, gingivitis, periodontitis, fungal stomatitis, aphte, canker ulceration, oral mucositis, leukoplakia, cheilitis, cystic fibrosis, blood clots, immune disorders, an autoimmune disease, cancer, herpes, herpes zoster or varicella of a mammal.

7. The composition of any of the preceding claims, wherein the composition is a composition to be applied by means of injection, ingestion, or intradermal penetration or by transdermal, transmucosal, or topical application.

8. The composition of any of the preceding claims, wherein the composition is incorporated in a dressing, a gel, a foam, or an intradermal patch.

## Patentansprüche

1. Enzymhaltige Gelzusammensetzung, umfassend mindestens ein proteolytisches Enzym, das aus antarktischem Krill erhalten wurde, mindestens ein Geliermittel und mindestens ein Verdünnungsmittel, das ein Polyol umfasst, zur Verwendung als Medikament, wobei die enzymatische Konzentration des Enzyms 0,01 U/ml bis 10 U/ml beträgt, wobei eine Einheit (U) der Aktivität die Freisetzung von 1 µmol Tyrosin aus einem Tyrosinstandard pro Minute bei 35 °C bewirkt, wobei das Polyol aus der Gruppe ausgewählt ist, die aus Propylenglykol, Ethylenglykol, Butylenglykol, Glycerin und Mischungen davon besteht, wobei das Geliermittel aus der Gruppe ausgewählt ist, die aus Glycerylpolymethacrylat, Glycerylacrylat und einem Glycerylacrylsäure-Copolymer besteht, wobei das Verdünnungsmittel bis zu 5 Gew.-% Wasser umfasst.

2. Zusammensetzung nach Anspruch 1, wobei die Gelzusammensetzung etwa 0,1 U/ml bis etwa 6 U/ml des Krill-Enzyms enthält.

3. Zusammensetzung nach einem der vorhergehenden Ansprüche, die ferner eine wirksame Menge einer Verbindung enthält, die aus der Gruppe ausgewählt ist, die aus mindestens einem Beschleuniger, insbesondere Harnstoff, mindestens einem Metallion und einer Mischung davon besteht, um die enzymatische Aktivität der Zusammensetzung zu erhöhen.

4. Zusammensetzung nach einem der vorhergehenden Ansprüche, wobei das Verdünnungsmittel bis zu 3 Gew.-%, insbesondere bis zu 2 Gew.-%, Wasser enthält.

5. Zusammensetzung nach einem der vorhergehenden Ansprüche, die ferner ein Konservierungsmittel, insbesondere Methylparaben, Ethylparaben, Propylparaben und/oder Polyhexa-Methylen-Biguanid, enthält.

6. Zusammensetzung nach einem der vorhergehenden Ansprüche zur Verwendung bei einem Säuger als topisches Mikrobiozid, zur Verhinderung einer rektalen oder vaginalen Übertragung von HIV, zum Schutz gegen andere sexuell übertragbare Krankheiten, insbesondere gegen Chlamydien oder Gonorrhoe, beim Gewebedebridement, Entfernen von devitalisiertem Gewebe bei einem nekrotischen Geschwür, Dekubitus, bei einer Verbrennung, bei plastischer Chirurgie oder anderen tiefen Gewebeläsionen, insbesondere Abszessen, Fisteln, Pleuritis, Empyem, Hämatothorax, Osteomyelitis, Otitis, Sinusitis oder topischen Geschwüren, insbesondere bei Lepra oder Leishmaniose, bei einer Narbenreduktion, zur Sterilisation oder zur Unterstützung von Transplantationen oder zur Verwendung bei der Behandlung von Hyperpigmentierung, einer Wunde, einer Infektion, einer nekrotischen Wunde, einer Hauterkrankung, insbesondere Akne oder Psoriasis, einer Pilzinfektion, einer bakteriellen Infektion, einer viralen Infektion, einer parasitären Infektion, einer Hämorrhoide, von Hornhautvernarbung, Zahnbelag, Gingivitis, Parodontitis, Pilzstomatitis, einer Aphte, Mundgeschwürentwicklung, Mundschleimhautentzündung, Leukoplakie, Cheilitis, Mukoviszidose, Blutgerinnseln, Immunstörungen, einer Autoimmunerkrankung, von Krebs, Herpes, Herpes Zoster oder Varizellen eines Säugers.

7. Zusammensetzung nach einem der vorhergehenden Ansprüche, wobei die Zusammensetzung eine Zusammensetzung ist, die durch Injektion, Einnahme oder intradermale Penetration oder durch transdermale, transmukosale oder topische Anwendung anzuwenden ist.

8. Zusammensetzung nach einem der vorhergehenden Ansprüche, wobei die Zusammensetzung in einem Verband, einem Gel, einem Schaum oder einem intradermalen Pflaster enthalten ist.

## Revendications

1. Composition de gel contenant une enzyme comprenant au moins une enzyme protéolytique obtenue à partir de krill antarctique, au moins un agent gélifiant et au moins un diluant comprenant un polyol pour utilisation comme médicament, dans laquelle la concentration enzymatique de l'enzyme va de 0,01 U/ml à 10 U/ml, où une unité (U) d'activité provoque la libération de 1 µmole de tyrosine provenant d'un étalon de tyrosine, par minute à 35 °C, dans laquelle ledit polyol est sélectionné parmi le groupe constitué du propylène glycol, de l'éthylène glycol, du butylène glycol, du glycérol et de mélanges de ceux-ci, dans laquelle ledit agent gélifiant est sélectionné parmi le groupe constitué du polyméthacrylate de glycéryle, de l'acrylate de glycéryle et d'un copolymère de glycéryle et d'acide acrylique, dans laquelle ledit diluant comprend jusqu'à 5 % en poids d'eau.

2. Composition selon la revendication 1, dans laquelle la composition de gel contient d'environ 0,1 U/ml à environ 6 U/ml de ladite enzyme de krill.

3. Composition selon l'une quelconque des revendications précédentes, comprenant en outre une quantité efficace d'un composé sélectionné parmi le groupe constitué d'au moins un accélérateur, notamment l'urée, d'au moins un ion métallique et d'un mélange de ceux-ci pour amplifier l'activité enzymatique de la composition.

4. Composition selon l'une quelconque des revendications précédentes, dans laquelle ledit diluant comprend jusqu'à 3 %, notamment jusqu'à 2 %, en poids d'eau.

5. Composition selon l'une quelconque des revendications précédentes, comprenant en outre un conservateur, notamment le méthylparabène, l'éthylparabène, le propylparabène et/ou le polyhexaméthylènebiguanide.

6. Composition selon l'une quelconque des revendications précédentes pour utilisation avec un mammifère en tant que microbiocide topique, dans la prévention de la transmission rectale ou vaginale du VIH, dans la protection contre d'autres troubles sexuellement transmissibles, notamment contre la chlamydia ou la gonorrhée, dans le débridement de tissu, le retrait de tissu dévitalisé en cas d'ulcère nécrotique, de décubitus, de brûlure, de chirurgie plastique ou d'autres lésions de tissu profond, notamment d'abcès, de fistules, de pleurésie, d'empyème, d'hémothorax, d'ostéomyélite, d'otite, de sinusite ou d'ulcères topiques, notamment en cas de lèpre ou de leishmaniose, dans la réduction de cicatrice, dans la stérilisation ou dans l'aide aux greffes, ou pour utilisation dans le traitement de l'hyperpigmentation, d'une plaie, d'une infection, d'une plaie nécrotique, d'un trouble cutané, notamment l'acné ou le psoriasis, d'une infection fongique, d'une infection bactérienne, d'une infection virale, d'une infection parasitaire, d'une hémorroïde, de la taie de la cornée, de la plaque dentaire, de la gingivite, de la parodontite, de la stomatite fongique, de l'aphte, de l'ulcération buccale, de la mucosité orale, de la leucoplasie, de la chéilite, de la mucoviscidose, de caillots sanguins, de troubles immunitaires, d'une maladie auto-immune, du cancer, de l'herpès, du zona ou de la varicelle d'un mammifère.

7. Composition selon l'une quelconque des revendications précédentes, dans laquelle la composition est une composition devant être appliquée au moyen d'une injection, ingestion ou pénétration intradermique ou par application transdermique, transmuqueuse ou topique.

8. Composition selon l'une quelconque des revendications précédentes, dans laquelle la composition est incorporée dans un pansement, un gel, une mousse ou un patch intradermique.
